# EUROPEAN PATENT SPECIFICATION

(11) **EP 1 750 127 B1**
(45) Date of publication and mention of the grant of the patent: **20.07.2011**
(21) Application number: 05741159.7
(22) Date of filing: 18.05.2005
(51) Int. Cl.: G01N 33/543

(54) **Method of affinity separation.**
Verfahren zur Affinitätstrennung.
Procédé de separation d'affinité.

(30) Priority: 24.05.2004 JP 2004153244; 11.05.2005 JP 2005138551
(43) Date of publication of application: 07.02.2007
(73) Proprietor: Shiseido Company, Limited, Tokyo 104-8010 (JP)
(72) Inventor: MIYAZAWA, K., SHISEIDO RESEARCH CTR. SHIN-YOKOHAMA, Yokohama-shi, Kanagawa 224-8558 (JP); MAENO, Katsuyuki, SHISEIDO RES. CTR. SHIN-YOKOHAMA, Yokohama-shi, Kanagawa 224-8558 (JP)
(74) Representative: Merkle, Gebhard
(86) International application number: PCT/JP2005/009085
(87) International publication number: WO 2005/114193

(56) References cited:
- WO-A1-02/18953
- JP-A- 8 333 421
- JP-A- 10 114 800
- JP-A- 11 005 817
- JP-A- 2001 228 149
- PARK J ET AL: "EVALUATION OF 2-METHACRYLOYLOXYETHYL PHOSPHORYLCHOLINE POLYMERIC NANOPARTICLE FOR IMMUNOASSAY OF C-REACTIVE PROTEIN DETECTION" ANALYTICAL CHEMISTRY, AMERICAN CHEMICAL SOCIETY. COLUMBUS, US, vol. 76, no. 9, 1 May 2004 (2004-05-01), pages 2649-2655, XP001196758 ISSN: 0003-2700
- KONNO T ET AL: "Conjugation of enzymes on polymer nanoparticles covered with phosphorylcholine groups" BIOMACROMOLECULES, ACS, WASHINGTON, DC, US, vol. 5, no. 2, 1 March 2004 (2004-03-01), pages 342-347, XP003006077 ISSN: 1525-7797
- MARTIN LENORE M: "Facile reduction in the synthesis of phosphorylcholine affinity columns" TETRAHEDRON LETTERS, vol. 37, no. 44, 1996, pages 7921-7928, XP002492562 ISSN: 0040-4039
- SAKAKI S ET AL: "WATER-SOLUBLE 2-METHACRYLOYLOXYETHYL PHOSPHORYLCHOLINE COPOLYMER AS A NOVEL SYNTHETIC BLOCKING REAGENT IN IMMUNOASSAY SYSTEM" POLYMER JOURNAL, SOCIETY OF POLYMER SCIENCE, TOKYO, JP, vol. 32, no. 8, 1 January 2000 (2000-01-01), pages 637-641, XP009055804 ISSN: 0032-3896
- TOMOHIRO TAKENORI ET AL: "Total analysis and purification of cellular proteins binding to cisplatin-damaged DNA using submicron beads.", BIOCONJUGATE CHEMISTRY 2002 MAR-APR LNKD- PUBMED:11906250, vol. 13, no. 2, March 2002 (2002-03), pages 163-166, ISSN: 1043-1802

## Description

### TECHNICAL FIELD

The present invention relates to an affinity separation method. More specifically, it relates to an affinity separation method using affinity particles utilizing organic particles that allows easy and highly precise separation of the target substance. The affinity particles used in the method of the present invention are very useful in various separation, purification, and testing methods including latex agglutination methods and immunoprecipitation methods that allow easy and highly sensitive detection of the target substance.

### BACKGROUND ART

Conventionally, column chromatography has been used for separation and purification of biological substances. However, column separation has some fatal problems as described in the following (1) to (3) :
(1) Many kinds of columns have to be used to obtain the target substance, resulting in a poor purification efficiency.
(2) A verification test is required to make sure the target substance is contained in the fractionated ingredients, which means purification is time consuming.
(3) Because of the large purification loss, a large quantity of the sample is required.

On the other hand, for separation and purification of the target substances, affinity particles and affinity columns supporting ligands are used (Patent Document 1 and Patent Document 2).

However, separation and purification using affinity columns have the following problems:
(1) The desired target substance is not selectively separated. That is, in addition to the target substance captured by the ligand, unwanted substances are also adsorbed onto the column.
(2) The capture efficiency is low, which means a large quantity of the liquid sample is required.

The affinity separation method in which affinity particles are dispersed in a liquid sample for separation uses agarose and such (Non-patent Document 1), but this method has a problem in that the desired target substance is not selectively separated. That is, in addition to the target substance captured by the ligand, unwanted substances are also adsorbed onto the affinity particles.

In addition to the aforementioned fatal problems, the affinity particles made of organic particles have a problem in that the organic particles tend to aggregate in samples having a high salt concentration. Because of this, the measurement has to be conducted with a diluted sample.

Patent Document 1: Japanese Patent Publication H8-26076
Patent Document 2: Japanese Patent Laid-Open H2002-511141 bulletin
Non-patent Document 1: Bioconjugate Chem. ; 2002; 13 (2) ; 163-166

Park J et al, "Evaluation of 2-methacryloyloxyethyl phosphorylcholine polymeric nanoparticle for immunoassay of C-reactive protein detection", Analytical Chemistry, American Chemical Society, Columbus, US, Vol. 76, No. 9, May 1, 2004 disclose the preparation of novel 2-methacrylyoloxyethyl phosphorylcholine polymeric nanoparticles (MPC-PNP).

Konno T et al, "Conjugation of enzymes on polymer nanoparticles covered with phosphorylcholine groups", Biomacromolecules, ACS, Washington, DC, US, Vol. 5, No. 2, March 1, 2004 disclose a bioconjugation of enzymes on polymer nanoparticles covered with bioinert phosphorylcholine groups.

Martin Lenore M, "Facile reduction in the synthesis of phosphorylcholine affinity columns", Tetrahedron Letters, Vol. 37, No. 44, 1996, pages 7921-7928 discloses the reduction of p-nitrophenylphosphorylcholine to the amine utilizing amonium formate and 10 % Pd/C in methanol, followed by in situ generation of the diazonium salt resulted in the synthesis of p-diazophenylphosphorylcholine.

JP 10 114800 A discloses a phosphorylcholine group-containing polymer composed of a polymer obtained by polymerizing a polymerizable component containing 2-methacryloyloxyethyl-2'-(trimethyl ammonio)ethyl phosphate which is adsorbed on an immunologically active substance immobilized stationary phase.

Sakaki S et al, "Water-soluble 2-methacryloyloxyethyl phosphorylcholine copolymer as a novel synthetic blocking reagent in immunoassay system", Polymer Journal, Society of Polymer Science, Tokyo, JP, Vol. 32, No. 8, January 1, 2000 disclose the development of a novel synthetic blocking reagent for the enzyme-linked immunosorbent assay (ELISA) method.

Takenori Tomohiro et al. (Bioconjugate chemistry 2002, volume 13(2), page 163-166) discloses an affinity method using latex beads synthesized by copolymerization of styrene and glycidyl methacrylate (GMA). The GMA makes the surface hydrolic, which significantly reduces nonspecific absorption of proteins to the beads.

### DISCLOSURE OF INVENTION

### [Problem that the present invention aims to solve]

The present invention aims to solve the afore mentioned problems and provides testing methods using affinity particles composed of organic particles that are used for various separation, purification.

### [Means to solve the Problem]

That is, the present invention provides a method of affinity separation according to claims 1-5.

When the affinity particles used in the method of the present invention are used for detection of antibodies and protein, such as in the immunoprecipitation method and the latex agglutination method, the recovery process (2) is not required; detection can be done easily by visually observing changes in the dispersion state.

### [Effects of the invention]

The affinity particles used in the method of the present invention use ligands to capture only a certain target substance (the substance desired to be separated) and suppresses adsorption of other substances onto the particles, resulting in a very high separation selectivity. Also, due to their superior dispersion properties, the target substance can be easily and accurately separated without causing aggregation even in a sample having various salts, such as serum.

That is, the target substance separation method of the present invention can effectively and easily separate the target substance to be separated in a short amount of time. Since substances have a tendency to adsorb onto foreign substances, conventional affinity particles have difficulties efficiently isolating only the target substance; however, it is possible to very efficiently prevent non-specific adsorption of the target substance to the affinity particles and thus increase the purification yield by modifying the particle surface with phosphorylcholine groups.

Also, phosphorylcholine groups are extremely hydrophilic and they also improve the dispersion properties of the affinity particles in a liquid sample containing water.

Furthermore, conventional particles tend to aggregate in the presence of salts and therefore the purification efficiency decreases when the target substance is to be isolated from serum because of aggregation due to various salts in serum; however, the affinity particles of the present invention don't aggregate significantly even under the presence of salts, which makes it possible to recover the target substance efficiently.

### BRIEF DESCRIPTION OF DRAWINGS

FIG. 1 is a schematic showing the difference between the protein capture selectivity of the affinity particles of the present invention and conventional affinity particles.
FIG. 2 shows a chemical structure formula and an NMR spectrum of the chemical compound prepared in Synthesis example 1.
FIG. 3 shows a chemical structure formula and an NMR spectrum of the chemical compound prepared in Synthesis example 2.
FIG. 4 shows a graph comparing the particle size distribution of conventional affinity particles in water and in a saline solution.
FIG. 5 shows a graph comparing the particle size distribution of the affinity particles of the present invention in water and in a saline solution.
FIG. 6 is a graph comparing the protein adsorption level of the styrene-glycidyl methacrylate particles and PC particles (A) prepared in Reference example 1.
FIG. 7 is a graph comparing the protein adsorption level of the styrene-glycidyl methacrylate particles and PC particles (B) and (C) prepared in Reference example 2.
FIG. 8 is a graph comparing the protein adsorption level of the agarose beads and PC particles (D) prepared in Reference example 3.
FIG. 9 is a graph comparing the antibody selectivity of the affinity particles of Example 1.
FIG. 10 is a graph comparing the antibody selectivity of the affinity particles of Comparative example 1.

### BEST MODE FOR CARRYING OUT THE INVENTION

The present invention is described in detail below.

### "Organic particles"

The selection of the organic particles that constitute the affinity particles is not limited in particular in the present invention. "An organic particle" generally means any organic object having an average particle size of about 20 nm to 500 µm. Specific examples of such particles include synthetic particles whose polymer contains one, two or more types of monomer units chosen from a group consisting of styrene, glycidyl methacrylate, (meth)acrylic acid, N-alkylacrylamide, alkyl (meth)acrylate, aminoalkyl (meth)acrylate, and hydroxyalkyl (meth)acrylate, or organic particles composed of agarose or sepharose. Hybrid particles having a core-shell structure in which the outer layer is organic and the inner particle is inorganic are also included.

Particularly preferable particles are those easily synthesized by means of emulsion polymerization, suspension polymerization, etc. ; examples include styrene-divinylbenzene copolymer, styrene-glycidyl methacrylate-divinylbenzene copolymer, acrylic acid-N-isopropyl acrylamide-methylene bisacrylamide copolymer, 2-hydroxy methacrylate-styrene-divinylbenzene copolymer, and 2-aminoethyl methacrylate-N-isopropyl acrylamide-methylene bisacrylamide copolymer.

Since the phosphorylcholine group represented by the aforementioned formula (1) and reactive groups or adsorptive groups that are capable of bonding with ligands are to be introduced onto the particle surface by means of covalent bonding, the surface should have reactive groups such as amino groups and hydroxyl groups

Also, the affinity particles having an average particle size of the organic particles of 20 nanometers to 500 µ m are preferable.
Examples include styrene-divinylbenzene copolymer, styrene-glycidyl methacrylate-divinylbenzene copolymer, acrylic acid-N-isopropyl acrylamide-methylene bisacrylamide copolymer, 2-hydroxy methacrylate-styrene-divinyl benzene copolymer, and 2-aminoethyl methacrylate-N-isopropyl acrylamide-methylene bisacrylamide copolymer.

### "Reactive groups or adsorptive groups to which the ligand can bind"

The selection is not limited as long as bonding with the ligand is possible. Preferable examples of the covalent bond form include an amide, ester, urethane, ether, secondary amine, urea bond, and disulfide bond. Therefore, reactive groups for which ligands can take the corresponding covalent bond forms are an amino group and hydroxyl group. Also, for the adsorption form, preferable are an avidin-biotin, metal-chelating compound, etc. Therefore, adsorptive groups for which ligands can take the corresponding adsorptive forms are preferable; examples include avidin, biotin, and chelating compounds.

### "Ligands"

In the present invention, a "ligand" means a substance that binds specifically to a certain target substance; examples include various antibodies, antigens, enzymes, substrates, receptors, peptides, aptamers, protein A, protein G, avidin, biotin, chelating compounds, and various metal ions Examples of the various antibodies include IgG, IgM, IgA, IgD, IgE, IgY, and polysaccharides, examples of enzymes include glutathione-S-transferase, examples of substrates include glutathione, examples of receptors include hormone receptors, cytokine receptors, examples of ligands include lectin, examples of chelating compounds include nitrile triacetate, and examples of various metal ions include Ni²⁺, Co²⁺, Cu²⁺, Zn²⁺, and Fe³⁺.

### A method of preparing the affinity particles of the present invention"

Since the essence of the present invention is to have the phosphorylcholine group represented by formula (1) covalently bonded through amino groups or hydroxyl groups on the surface of organic particles and also for the organic particles to have reactive groups or adsorptive groups capable of bonding to ligands having specific affinity to a certain target substance that are directly present on their surface by means of covalent bonding or adsorption, there is no limitation on the selection of the preparation method; bonding can be done with any means.
However, as mentioned earlier, this does not include methods in which a polymer already having the phosphorylcholine group and reactive groups or adsorptive groups capable of bonding to ligands is used to simply coat the particle surface without chemical bonding. This is because the coating polymer can peel off and/or there may be an influence from the coating polymer.

The affinity particles used in the method of the present invention can be prepared with the following method, for example.
Step 1: The phosphorylcholine group represented by the following formula (1) and reactive groups or adsorptive groups capable of bonding to ligands are introduced onto the particles. The selection of the reactive group or adsorptive group is an amino group and an hydroxyl group.
Step 2: The phosphorylcholine group represented by formula (1) and the ligand are bonded to the reactive group or adsorptive group introduced onto the particles. Any chemical structure (spacer) can exist between the phosphorylcholine group or ligand and the reactive group or adsorptive group. Examples of such arbitrary spacers include a methylene chain, oxyethylene chain, as well as an alkylene chain containing one or a plurality of amino groups.

### "When the reactive group or adsorptive group on the particle surface is an amino group"

Step 1: Amino groups are introduced to any particle by using a prior art method or a method that will be developed in the future. Amino groups are directly introduced onto the particle surface. The amino group can be a primary amine or a secondary amine.
Step 2: An aldehyde derivative or hydrate derivative obtained by the oxidative cleavage reaction of glycerophosphorylcholine is used in a reductive amination reaction to directly add phosphorylcholine groups to the surface of the particle having amino groups.
Not all the amino groups are bonded with the phosphorylcholine group (the reaction level is controlled) so that the remaining amino groups are available as substituents for the ligand to bind to.
Or, a carboxyl derivative obtained by the oxidative cleavage of glycerophosphorylcholine is used in an amidation reaction to directly add phosphorylcholine groups to the surface of the particles having amino groups. Not all the amino groups are bonded with the phosphorylcholine group (the reaction level is controlled) so that the remaining amino groups are available as substituents for the ligand to bind to.

### "A method of introducing amino groups onto the particle surface"

Examples of a prior art method for introducing amino groups to the particles (step 1) follow:

### 1. Introduction of amino groups by means of a surface reaction via a plasma treatment

Amino groups are introduced to the particle surface by means of a low temperature plasma in a nitrogen gas atmosphere. Specifically, the particles are put into a plasma reactor vessel and, after a vacuum pump is used to form a vacuum in the reactor vessel, nitrogen gas and hydrogen gas are introduced. Amino groups can be then introduced onto the particle surface by means of glow discharge. It is also possible to mechanically turn the plasma-treated organic material into particles. References related to the plasma treatment are shown below:
1. M. Muller, C. oehr Plasma aminofunctionalisation of PVDF microfiltration membranes: comparison of the in plasma modifications with a grafting method using ESCA and an amino-selective fluorescent probe Surface and Coatings Technology 116-119 (1999) 802-807
2. Lidija Tusek, Mirko Nitschke, Carsten Werner, Karin Stana-Kleinschek, Volker Ribitsch Surface characterization of NH3 plasma treated polyamide 6 foils Colloids and Surfaces A: Physicochem. Eng. Aspects 195 (2001) 81-95
3. Fabienne Poncin-Epaillard, Jean-Claude Brosse, Thierry Falher Reactivity of surface groups formed onto a plasma treated poly (propylene) film Macromol. Chem. Phys. 200. 989-996 (1999)

### 2. Introduction of amino groups by means of a surface modifier

The surface of the organic particles such as alkoxysilyl group-containing particles is treated with a surface modifier having amino groups, such as alkoxysilane, chlorosilane, and silazane.
For example, alkoxysilyl group-containing particles are treated with 3-aminopropyltrimethoxysilane, which has a primary amino group, to introduce amino groups. Specifically, the 3-trimethoxysilylpropyl-1-methacrylate-methyl methacrylate-divinylbenzene copolymer particles are soaked in a mixed solution of water and 2-propanol, and, after adding 3-aminopropyltrimethoxysilane, the temperature is raised to 50 °C and the reaction is carried out for six hours. After cooling down to room temperature, the aforementioned polymer is rinsed with methanol and dried to obtain particles that have amino groups directly introduced onto the aforementioned copolymer particles.

### 3. Introduction of amino groups by means of the silicone vapor phase treatment (Refer to Japanese Patent Publication No. H1-54379, Japanese Patent Publication No. H1-54380 bulletin, and Japanese Patent Publication No. H1-54381 bulletin.)

The particle surface is treated with 1.3.5.7-tetramethylcyclotetrasiloxane and then Si-H groups introduced onto the surface are reacted with monomers having an amino group to obtain an aminated surface. For example, styrene-divinylbenzene particles and 1.3.5.7-tetramethylcyclotetrasiloxane are put into a desiccator and an aspirator is used to deaerate it. The reaction is carried out for 16 hours at 80 °C, and the aforementioned particles are taken out and dried at 50 °C. The obtained particles are dispersed in ethanol, to which allylamine is added, and an ethanol solution of chloroplatinic acid is added, followed by two hours of stirring at 60 °C. After the reaction is completed, filtration, ethanol rinsing, and reduced-pressure drying are carried out to obtain aminated organic particles.
For the monomer to be used in this method, an amine-type monomer can be used. The amine-type monomer is not limited to allylamine as long as it has a reactive site such as polymerizable vinyl and acrylate, and an amino group. The amino group can be protected by a butoxycarbonyl group, benzyloxycarbonyl group or the like.
In addition to an amine-type monomer, a monomer having a functional group such as an epoxy group, to which an amino group can be easily introduced by means of, for example, a reaction with diamine, can be used as well.

### "A method for introducing phosphorylcholine groups onto the particles having amino groups"

Next, a method for introducing phosphorylcholine groups onto the aminated particle surface (step 2) is described below.
The particles are soaked in methanol, to which phosphatidylglyceroaldehyde is added, and [the mixture is] left alone for six hours at room temperature. Sodium cyanoborate is then added at 0 °C, followed by overnight heating and stirring, to add a phosphorylcholine group to an amino group. The particles are rinsed with methanol and dried to obtain particles that have phosphorylcholine groups directly on the surface. For the reaction solvent, protic solvents such as water, ethanol, and 2-propanol can be used in addition to methanol; the introduction rate tends to be higher when methanol is used.
Or, the particles are dispersed in a mixed solution of dimethylsulfoxide-water, to which N-hydroxysuccinimide, 1-ethyl-3-(3-dimethylaminopropyl) carbodiimide hydrochloride, and carboxymethyl phosphorylcholine dissolved in a mixed solution of dimethylsulfoxide-water is added. After stirring for 6 hours at room temperature the particles are thoroughly rinsed with water and then they are dried to obtain particles having phosphorylcholine groups directly on the surface. In addition to what is mentioned above, aprotic solvents such as N,N'-dimethylformamide, tetrahydrofuran, and acetonitrile can be used as the reaction solvent. Or, carboxymethyl phosphorylcholine and thionyl chloride are reacted and the obtained acid chloride is reacted with the particles under anhydrous conditions using a solvent such as N,N'-dimethylformamide and acetonitrile, the particles are then thoroughly rinsed with water and dried to obtain particles having phosphorylcholine groups directly on the surface. This method also allows an efficient reaction with hydroxyl groups on the surface, and therefore is effective when particles are composed of a polysaccharide such as agarose and sepharose or 2-hydroxyethyl (meth)acrylate.

Shown below is a scheme in which amino groups are introduced onto organic particles having alkoxysilyl groups by using 3-aminopropyl trimethoxysilane as the surface modifier and then phosphorylcholine groups (abbreviated as PC) are introduced.

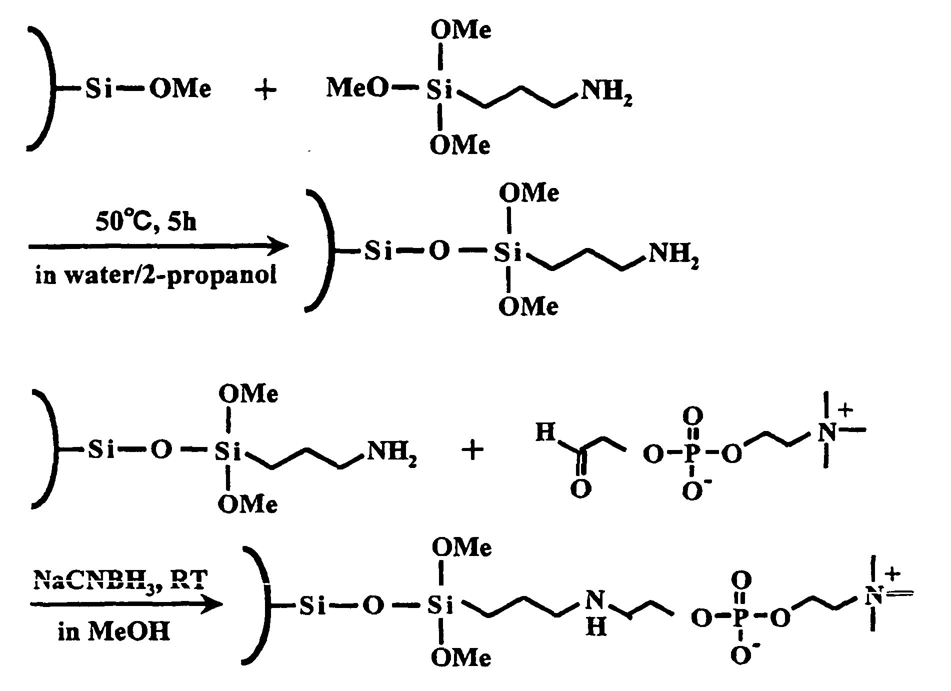

As described above, the particles directly having phosphorylcholine groups on the surface can be obtained by a method in which particles having amino groups are prepared and then a reductive amination reaction with a hydrate derivative or aldehyde derivative obtained by the oxidative cleavage reaction of glycerophosphorylcholine is used to directly add phosphorylcholine groups to the particle surface.
This method has the following great advantages: the introduction rate of the phosphorylcholine group is high, and the surface of various organic particles can be modified.

In the aforementioned method, the compound containing the aldehyde derivative obtained by the oxidative cleavage reaction of glycerophosphorylcholine is obtained by oxidative cleavage of the prior art glycerophosphorylcholine group by means of a prior art method, which is a very easy step. This reaction uses periodic acid or periodate to oxidize 1,2-diol to open the bond and obtain two aldehyde derivatives; in this particular method, a phosphorylcholine aldehyde derivative and formaldehyde are produced. The reaction is usually carried out in water or in an organic solvent containing water. The reaction temperature is between 0 °C and room temperature. The aldehyde derivative may go through an equilibrium reaction in water to become a hydrate, but this does not affect the subsequent reaction with the amine. A scheme for preparing a monofunctional aldehyde derivative containing a phosphorylcholine group is described below.

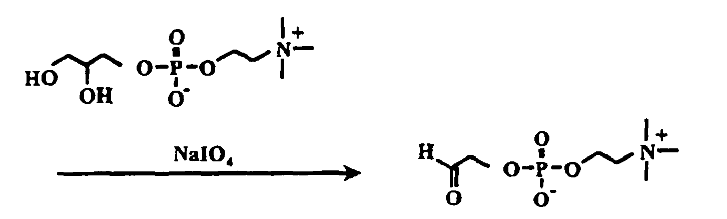

The reductive amination reaction for bonding the aldehyde derivative (or hydrate derivative) obtained by the oxidative cleavage reaction of glycerophosphorylcholine to the amino groups of the particles can be carried out easily by stirring both of them in a solvent. This reaction is carried out by dissolving or dispersing these two in water or alcohol (a third organic solvent ingredient can be mixed in, too) to form an imine and reducing it with a reducing agent to obtain a secondary amine. For the reducing agent, a mild reducing agent such as sodium cyanoboronate is preferable, but other reducing agents can be used as long as the phosphorylcholine is stable. The reaction is usually carried out at 0 °C to room temperature, but heating may be added depending on the situation.

It is also possible to react any amount of the compound represented by formula (2) to the aforementioned amino groups and leave the remaining amino groups as reactive groups or adsorptive groups to which ligands can bind. 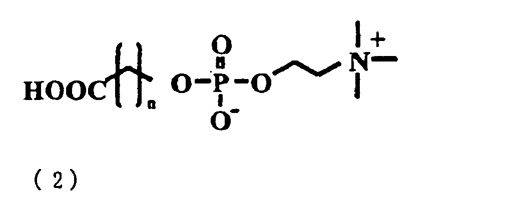 n denotes an integer 1-12.

### "Reactive groups or adsorptive groups to which ligands can bind"

Not all the amino groups are bonded with the phosphorylcholine group (the reaction level is controlled) so that the remaining amino groups are available as reactive groups or adsorptive groups for the ligand to bind to. These particles are the affinity particles described in claim 2, i.e. the particles that are organic particles directly having on their surface the phosphorylcholine groups represented by formula (1) and reactive groups or adsorptive groups to which the ligand can bind. When the ligand is bonded to these remaining amino groups, the affinity particles described in claim 3, i.e. the particles directly having the phosphorylcholine group represented by formula (1) and the ligand on their surface, are obtained.
The product form of the affinity particles described in claim 2 is such that the user can bind any ligand to the particles depending on the substance to be captured (target substance). The product form of the affinity particles described in claim 3 is such that the ligand is already bonded. The affinity particles described in claim 1 are affinity particles having at least the phosphorylcholine group of formula (1) on the particle surface and their product form is such that the user can bind any ligand to them depending on the substance to be captured (target substance), regardless of the presence or absence of the ligand or reactive group or adsorptive group that can bind to it. Affinity particles of any form are included as long as the phosphorylcholine group of formula (1) is present on the particle surface; for example, the forms described in claim 2 and claim 3 are included as well.

In the aforementioned reaction, leaving some amino groups as reactive groups or adsorptive groups to which the ligand can bind can be made possible, for example, by adjusting the reaction quantity or by a competitive reaction of 3-aminopropyl trimethoxysilane and 3-aminopropyl trimethoxysilane to which the phosphorylcholine group is introduced.
It is also possible to react this amino group with a compound having any functional group and use this functional group as the reactive group or adsorptive group to which the ligand can bind. Examples include glutaraldehyde, alkyl diimidate, acyl azides, and isocyanates.

In a scheme in which 3-aminopropyl trimethoxysilane is used for the aforementioned surface modifier, it is also possible to adjust the reaction quantity of the surface modifier to leave some hydroxyl groups (OH) on the particle surface and use these remaining OH groups as reactive groups or adsorptive groups to which the ligand can bind.

### "A method of binding the ligand to the particles having amino groups"

When the ligand is a protein, one aldehyde group of glutaraldehyde is reacted with an amino group on the organic particle and the other aldehyde group is reacted with an amino group in the protein, thus binding the protein.

### "When the reactive group or adsorptive group on the particle surface is a hydroxyl group"

When hydroxyl groups are present on the organic particles, no reactive group or adsorptive group to which the ligand can bind, such as amino groups as mentioned above, needs to be introduced; the hydroxyl groups (OH) present on the particle surface are used as they are to introduce the phosphorylcholine group and the ligand or reactive groups or adsorptive groups to which the ligand can bind. The affinity particles of the present invention are preferably prepared with this method.

### "A method for introducing phosphorylcholine groups onto the particles having hydroxyl groups"

A chemical bond is formed by dehydration of the hydroxyl group on the particle surface and Si-OMe of the compound of the following formula (3) or (4). This chemical reaction proceeds very easily and quantitatively in most organic solvents if heating and refluxing are provided. Chemically and physically very stable phosphorylcholine groups can be introduced by means of this dehydration reaction, which is preferable. The phosphorylcholine group-containing compound represented by the following formula (3) or (4) is a new compound. 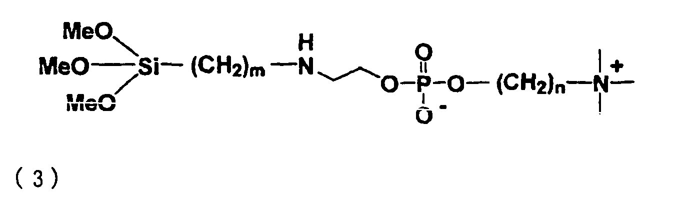 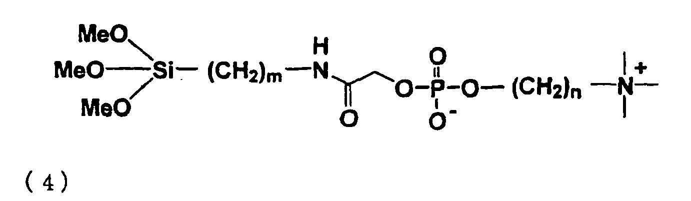 In this formula, m denotes 2-6 and n denotes 1-4. OMe can be replaced by OEt or Cl. Up to two of the OMe's, OEt's, or Cl's to be bonded to Si can be replaced by a methyl group, ethyl group, propyl group, isopropyl group, or isobutyl group.

### A method of preparing the phosphorylcholine group-containing chemical compound of formula (3)"

The phosphorylcholine derivative shown in the following formula (5) is dissolved in distilled water. The phosphorylcholine derivative of the following formula (5) is a prior art chemical compound and commercially available.

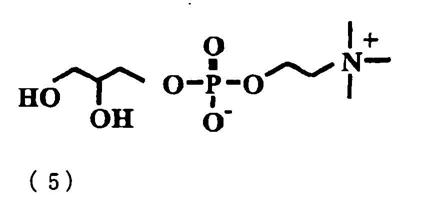

An aqueous solution of the chemical compound of formula (5) is cooled in an ice water bath; then sodium periodate is added, followed by five hours of stirring. The reaction fluid is concentrated under reduced pressure and dried under reduced pressure; methanol is used to extract a phosphorylcholine derivative having an aldehyde group shown in the following formula (6).

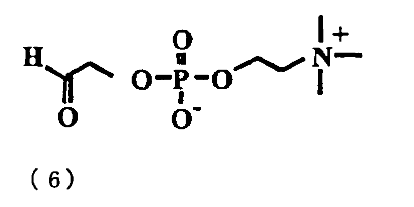

0.5 equivalents of 3-aminopropyltrimethoxysilane is added to the methanol solution of formula (6). This mixed solution is stirred for a prescribed amount of time at room temperature and cooled with ice; an appropriate amount of sodium cyanohydroborate is then added and the temperature is returned back to room temperature, followed by 16 hours of stirring. During this time dry nitrogen is continued to be fed through the reaction vessel. After filtering the precipitate, a methanol solution of formula (3) is obtained.

The procedure described above can be carried out in the same way even when m and n in the chemical compounds represented by formula (3) change. The procedure shown here is for m = 3 and n = 2. The reaction solvent is not limited in particular; in addition to methanol, which was mentioned above, water, alcohols such as ethanol, propanol, and butanol, and aprotic solvents such as DMF and DMSO can be used. Dehydrated solvents are preferable to prevent polymerization during the reaction; of these, dehydrated methanol is particularly preferable.
If a methoxy group (OMe) in formula (3) is replaced by an ethoxy group (OEt), then the reaction is carried out by using ethanol instead of methanol; if it is replaced by Cl, then dimethylformamide or dimethylsulfoxide is used instead.
Furthermore, even when one or two of the OMe groups, OEt, or Cl's to be bonded to Si are replaced by a methyl group, ethyl group, propyl group, isopropyl group, or isobutyl group, the preparation can be carried out in exactly the same manner as described above.

### "A method of preparing the phosphorylcholine group-containing chemical compound of formula (4)"

An aqueous solution of the chemical compound of formula (5) is cooled in an ice water bath; sodium periodate and a catalytic amount of ruthenium trichloride are added, followed by three hours of stirring. The reaction fluid is concentrated under reduced pressure and dried under reduced pressure; methanol is used to extract a phosphorylcholine derivative (7) having a carboxyl group.

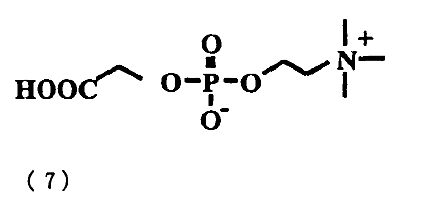

Next, 1.2 equivalents of thionyl chloride is added to formula (7) dispersed in acetonitrile or N,N-dimethylformamide and, after 30 minutes of stirring, 0.9 equivalents of 3-aminopropyltrimethoxysilane is added to the solution. This mixed solution is stirred for 4 hours at room temperature to obtain the chemical compound of formula (8).

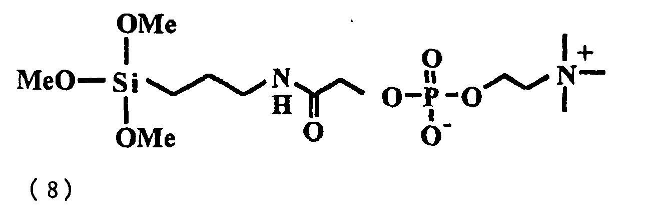

In addition to thionyl chloride, any reagent can be used for the aforementioned condensation reaction as long as it generates halogenated carboxylic acid; examples include phosphorus pentachloride, phosphorus oxychloride, phosphorus tribromide, and oxalyl chloride.

In addition to using the silane coupler of formula (8), it is also possible to have the compound of formula (7) directly react with the hydroxyl group. For example, sepharose beads are dispersed in anhydrous acetonitrile, to which an acetonitrile solution prepared by mixing the compound of formula (7) and 1.2 equivalents thionyl chloride, followed by overnight stirring, is added; after 3 hours of stirring, particles having the phosphorylcholine compound on the surface are obtained.

### Reactive groups or adsorptive groups to which ligands can bond"

Not all the hydroxyl groups are bonded with the phosphorylcholine group (the reaction level is controlled) so that the remaining hydroxyl groups are available as reactive groups or adsorptive groups for the ligand to bind to. These particles are the affinity particles described in claim 2, i.e. the particles that are organic particles directly having on their surface the phosphorylcholine groups represented by formula (1) and reactive groups or adsorptive groups to which the ligand can bond. When the ligand is bonded to these remaining hydroxyl groups, the affinity particles described in claim 3, i. e. the particles directly having the phosphorylcholine group represented by formula (1) and the ligand on their surface, are obtained.
The product form of the affinity particles described in claim 2 is such that the user can bind any ligand to them depending on the substance to be captured (target substance). The product form of the affinity particles described in claim 3 is such that the ligand is already bonded. The affinity particles described in claim 1 are affinity particles having at least the phosphorylcholine group of formula (1) on the particle surface and their product form is such that the user can bind any ligand to them depending on the substance to be captured (target substance), regardless of the presence or absence of the ligand or reactive group or adsorptive group that can bind to the ligand. Affinity particles of any form are included as long as the phosphorylcholine group of formula (1) is present on the particle surface; for example, the forms described in claim 2 and claim 3 are included as well.

### "A method of binding the ligand to the particles having hydroxyl groups"

When the ligand is a protein, hydroxyl groups on the particles are activated by using cyanogen bromide. Amino groups in the protein are reacted to these to bind the protein.
It is also possible to react this hydroxyl group with a compound having any functional group and use this functional group as the reactive group or adsorptive group to which the protein can bind.

### "When the reactive group or adsorptive group on the particle surface is a carboxyl 1 group"

Step 1: Carboxyl groups are introduced to any particle by using a prior art method or a method that will be developed in the future. Carboxyl groups are directly introduced onto the particle surface.
Step 2: It is also possible to react the phosphorylcholine-containing compound represented by formula (9) with the particles having carboxyl groups so as to form an acid amide bonding with the phosphorylcholine group and use the remaining carboxyl groups as reactive groups or adsorptive groups to which ligands can bind.
Not all the carboxyl groups are bonded with the phosphorylcholine group (the reaction level is controlled) so that the remaining carboxyl groups are available as reactive groups or adsorptive groups for the ligand to bind to.

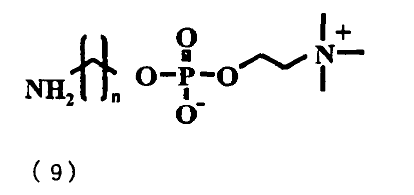

### "A method of introducing carboxyl groups onto the particle surface"

Examples of a prior art method for introducing carboxyl groups to the particles (step 1) follow:

### 1. Introduction of carboxyl groups by means of a surface modifier

The surface of the organic particles such as alkoxysilyl group-containing particles is treated with a surface modifier having carboxyl groups, such as alkoxysilane, chlorosilane, and silazane.

For example, organic particles having alkoxysilyl groups are treated with triethoxysilylpropyl succinate anhydrate to introduce carboxyl groups. Specifically, triethoxysilylpropyl succinate anhydrate is dissolved in N,N-dimethylformamide, to which distilled water and 4-dimethylaminopyridine is added, followed by stirring at room temperature for 16 hours to obtain a silane coupling agent having carboxylic acid. This reaction is a hydrolysis reaction of succinic acid anhydrate using 4-dimethylaminopyridine.

Organic particles having alkoxysilyl groups are soaked in a water-2-propanol mixed solution, to which the silane coupling agent having carboxylic acid is added, followed by heating up to 50 °C for 6 hours of reaction. After cooling down to room temperature, the organic particles are rinsed with methanol and dried to obtain particles that have carboxyl groups directly introduced onto organic particles.

### 2. Introduction of carboxyl groups by means of the silicone vapor phase treatment (Refer to Japanese Patent Publication No. H1-54379, Japanese Patent Publication No. H1-54380 bulletin, and Japanese Patent Publication No. H1-54381 bulletin.)

The particle surface is treated with 1. 3.5.7-tetramethylcyclotetrasiloxane and then Si-H groups introduced onto the surface are reacted with monomers having a carboxyl group to obtain a carboxylated surface.
For the monomer to be used in this method, a carboxyl-type monomer can be used. The selection of the carboxyl-type monomer is not limited as long as it has a reactive site such as a carboxyl group, polymerizable vinyl and acryl.

### "A method for introducing phosphorylcholine groups onto the particles having carboxyl groups"

Next, a method for introducing phosphorylcholine groups onto the carboxylated particle surface (step 2) is described below.
When particles having carboxyl groups on the surface are soaked in a solution of N-hydroxysuccinimide and 1-ethyl-3-(3-dimethylaminopropyl)-carbodiimide, the particle surface is coated with active ester groups. A solution of the phosphorylcholine derivative having an amino group represented by formula (9) is added to this to introduce phosphorylcholine groups.

### "Reactive groups or adsorptive groups to which ligands can bind"

Not all the carboxyl groups are bonded with the phosphorylcholine group (the reaction level is controlled) so that the remaining carboxyl groups are available as reactive groups or adsorptive groups for the ligand to bind to. These particles are the affinity particles described in claim 2, i.e. the particles that are organic particles directly having on their surface the phosphorylcholine groups represented by formula (1) and reactive groups or adsorptive groups to which the ligand can bond. When the ligand is bonded to these reactive or adsorptive groups to which the ligand can bind, the affinity particles described in claim 3, i.e. the particles directly having the phosphorylcholine group represented by formula (1) and the ligand on their surface, are obtained.
The product form of the affinity particles described in claim 2 is such that the user can bond any ligand to them depending on the substance to be captured (target substance). The product form of the affinity particles described in claim 3 is such that the ligand is already bonded. The affinity particles described in claim 1 are affinity particles having at least the phosphorylcholine group of formula (1) on the particle surface and their product form is such that the user can bind any ligand to them depending on the substance to be captured (target substance), regardless of the presence or absence of the ligand or reactive group or adsorptive group that can bind to the ligand. Affinity particles of any form are included as long as the phosphorylcholine group of formula (1) is present on the particle surface; for example, the forms described in claim 2 and claim 3 are included as well.

In the aforementioned reaction, leaving some carboxyl groups as reactive groups or adsorptive groups to which the ligand can bind can be made possible, for example, by adjusting the reaction quantity of the silane coupling agent having a carboxylic acid to which the phosphorylcholine group is introduced.
It is also possible to react this carboxyl group with a compound having any functional group and use this functional group as the reactive group or adsorptive group to which the ligand can bind.

### "A method of binding the ligand to the particles having carboxyl groups"

When the ligand is a protein, organic particles having carboxyl groups on the surface are soaked in a solution of N-hydroxysuccinimide and 1-ethyl-3-(3-dimethylaminopropyl)-carbodiimide to esterify the particle surface. Amino groups in the protein are reacted with these to bind the protein. It is also possible to react this hydroxyl group with a compound having any functional group and use this functional group as the reactive group or adsorptive group to which the ligand can bind.

### "A method of affinity separation of a target substance"

Using the affinity particles obtained as described above, the affinity separation of a target substance of the present invention is carried out.
The method of the present invention is a groundbreaking separation method for a target substance in that high precision separation can be easily done by using organic particles.
The method of the present invention contains the following 3 processes. The first process is omitted for the affinity particles to which the ligand is already bonded (claim 2) since this process has already been done for such particles.
1. The first process in which any ligand is chemically bonded to affinity particles that are characterized by having phosphorylcholine groups represented by the following formula (1) covalently bonded through amino groups or hydroxyl groups onto the surface of organic particles or affinity particles that are characterized by having phosphorylcholine groups represented by the following formula (1) covalently bonded through amino groups or hydroxyl groups onto the surface of organic particles and also by having reactive groups or adsorptive groups selected from amino groups and hydroxyl groups, that are capable of bonding with ligands having specific affinity with a certain target substance, covalently bonded or adsorbed onto the surface of organic particles.
   For example, 1 ml of a PBS solution of any ligand and affinity particles that are organic particles having the phosphorylcholine group represented by formula (1) covalently bonded onto their surface and reactive groups or adsorptive groups to which the ligand can bind covalently bonded or adsorbed on their surface are put into a 2 ml eppen tube, followed by gentle shaking at 4 °C for 30 minutes. This is centrifuged for 30 minutes at 15,000 rpm and the supernatant is discarded. The sample is cleaned by adding 1 ml of a PBS solution to it, gently shaking it, centrifuging it for 30 minutes at 15,000 rpm, and discarding the supernatant. This cleaning operation is repeated 3 times.
2. The second process in which the affinity particles prepared in the first process are dispersed in a liquid sample containing the target substance that is selectively captured by any ligand.
   For example, the affinity particles prepared in the first process are dispersed in a liquid sample containing the target substance that is selectively captured by any ligand, followed by gentle shaking for 30 minutes at 4 °C. This is centrifuged for 30 minutes at 15,000 rpm and the supernatant is discarded. The sample is cleaned by adding 1 ml of a PBS solution to it, gently shaking it, centrifuging it for 30 minutes at 15,000 rpm, and discarding the supernatant. This cleaning operation is repeated 3 times.
3. The third process in which the captured target substance is recovered from the separated affinity particles.
   For example, for the purpose of recovering the captured target substance from the affinity particles, 1 ml of an elution buffer is added, followed by gentle shaking for 30 minutes at 4 °C to elute the target substance from the particles, and the supernatant is recovered. 1 ml of a PBS solution is added to it, followed by gentle shaking and centrifugation for 30 minutes at 15,000 rpm, and the supernatant is recovered. This operation is repeated twice.

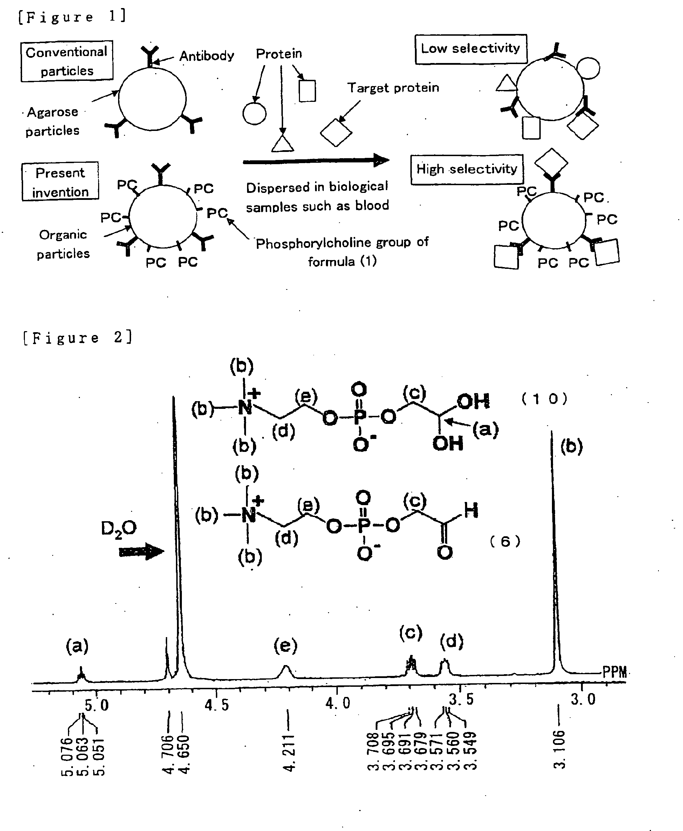

FIG. 1 is a schematic showing the differences between the target substance capture selectivity of the affinity particles of the present invention and conventional affinity particles.

### EXAMPLES

Next, the present invention is described in detail by referring to Examples. The present invention is not limited to these Examples. The phosphorylcholine group introduced onto the particle surface can be verified and quantified by means of the following method.

### <Quantification method>

The obtained particles were immersed in perchloric acid and heated up to 180 °C to be decomposed. The obtained solution was diluted with water, to which hexaammonium heptamolybdate tetrahydrate and L-ascorbic acid were added, followed by 5 minutes at 95 °C of color development time; the amount introduced was determined by means of the light absorption measurement at 710 nm. For the calibration curve, a sodium dihydrogen phosphate solution was used.

### "Synthesis example 1"

### "An aldehyde chemical compound containing phosphorylcholine groups"

1-α -glycerophosphorylcholine (6.29 g) was dissolved in 210 ml of distilled water and cooled in an ice water bath. Sodium periodate (10.23 g) was added, followed by five hours of stirring.
The reaction fluid was concentrated under reduced pressure and dried under reduced pressure; methanol was then used to extract the target substance. The structure of the compound is shown in the following chemical formula (6).
A 1H NMR spectrum of the compound of formula (6) is shown in FIG. 2. Since the compound of formula (6) is in equilibrium with formula (10) in water, the actual spectrum reflects both formula (6) and formula (10).

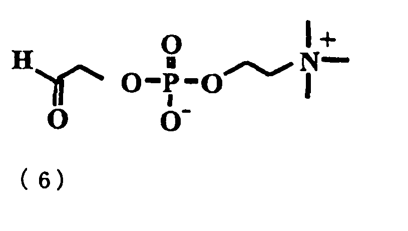

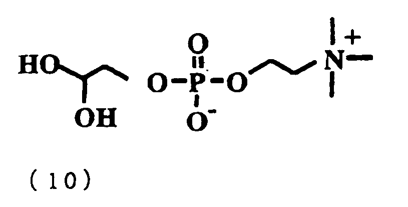

### "Synthesis example 2"

### "A carboxylic acid chemical compound containing phosphorylcholine groups"

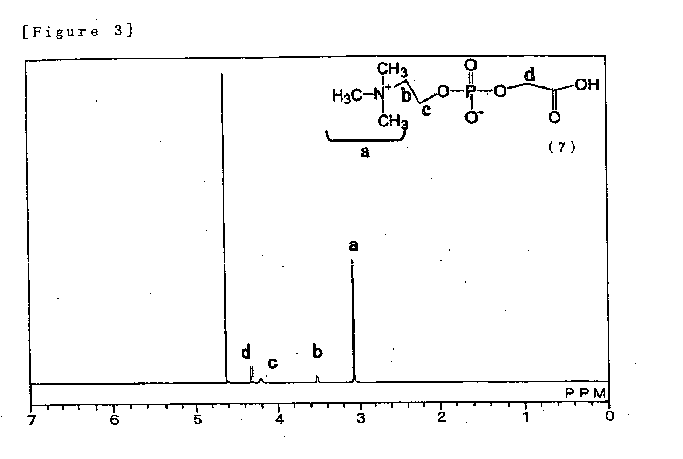

5 g of 1-α-glycerophosphorylcholine was dissolved in water (70 ml)/acetonitrile (30 ml). As the temperature was lowered with ice, 17 g of sodium periodate and 80 mg of ruthenium trichloride were added, followed by overnight stirring. After filtering the precipitate, concentration under reduced pressure and methanol extraction were carried out to obtain the target carboxymethyl phosphorylcholine represented by chemical formula (7).
A 1H NMR spectrum of the compound of formula (7) is shown in FIG. 3.

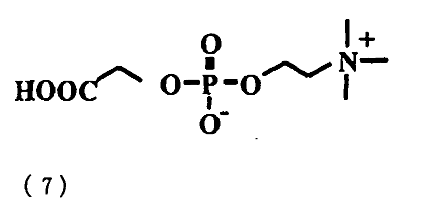

### "Reference example 1"

### "Styrene-glycidyl methacrylate particles"

3.6 g glycidyl methacrylate, 2.4 g styrene, and 0.08 g divinylbenzene were added to 220 ml of purified water that had been thoroughly deaerated by means of nitrogen substitution. 0.12 g of polymerization initiator V-60 was added, followed by stirring at 70 °C for 1 hour. An additional 0.6 g of glycidyl methacrylate was added, followed by overnight stirring at 70 °C. After cooling the temperature down to room temperature and purification by means of centrifugation (15,000 rpm x 30 minutes, 3 times), the target particles were obtained.

### "Styrene-glycidyl methacrylate particles with amino groups introduced"

1 g of styrene-glycidyl methacrylate particles were dispersed in 80 ml of purified water, to which 20 ml of 25% ammonia aqueous solution was added, followed by overnight heated stirring at 70 °C. The mixture was cooled down to room temperature and purified by means of centrifugation (17,000 rpm x 60 minutes, 3 times).

### "Phosphorylcholine-modified particles (PC particles (A))"

0.5 g of styrene-glycidyl methacrylate particles with amino groups introduced onto them were dispersed in 10 ml of methanol, to which 0.5 of the aldehyde derivative of Synthetic example 1 was added, followed by overnight stirring. 140 mg of sodium cyanoborate was added to the mixture in an ice bath, followed by stirring for 6 hours and purification by means of centrifugation (17,000 rpm x 60 minutes, 3 times) to obtain the PC particles (A).

### "Agglutination by using salt"

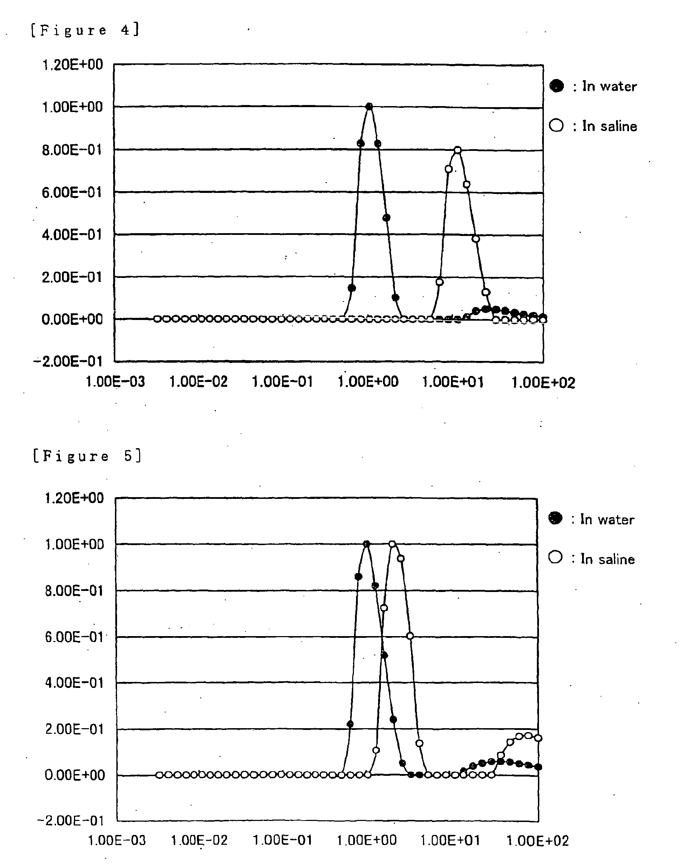

FIG. 4 and FIG. 5 show the particle size distribution of the PC particles and styrene-glycidyl methacrylate particles prepared in Reference example 1 in water and also in a saline solution (0.1 M aqueous solution).
FIG. 4 shows that styrene-glycidyl methacrylate particles generally used for the latex agglutination method using affinity particles have a significantly different particle size distribution in NaCl solution compared with that in water, which indicates that agglutination is taking place. On the other hand, FIG. 5 shows that the particle size change of the PC particles (A) in a saline solution is smaller compared with that in FIG. 4, indicating that agglutination due to salt occurs less. This indicates that the PC particles (A), due to modification with the phosphorylcholine of formula (1), are less influenced by interfering substances such as salt, which leads to a higher measurement accuracy.

### "Evaluation of suppression of non-specific adsorption of proteins"

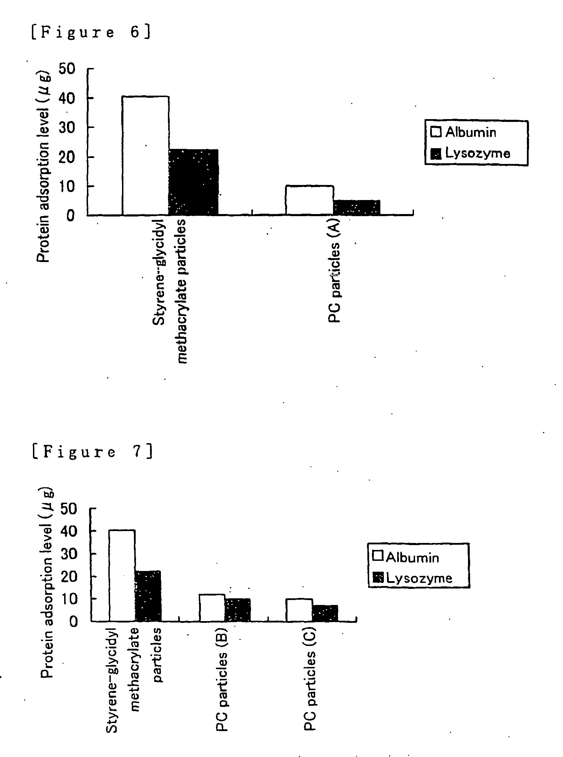

25 mg each of the styrene-glycidyl methacrylate particles and PC particles (A) prepared in Reference example 1 were sampled, to each of which 1 ml of distilled water was added, followed by 1 minute of an ultrasonic treatment. After removing distilled water by means of centrifugation, 1 mL of albumin (100 µg/mL) or lysozyme (100 µ g/mL) was added, followed by a 1 hour reaction at room temperature; after centrifugation (5,000 g), the supernatant was quantified with the Micro BCA method. The results are shown in FIG. 6. The PC particles (A), which had been treated with phosphorylcholine groups, showed significantly suppressed adsorption of both albumin and lysozyme compared with the styrene-glycidyl methacrylate particles. This indicates that the modification with the phosphorylcholine of formula (1) significantly reduces protein adsorption. Not only agglutination between particles but also the non-specific adsorption of various proteins onto the particles significantly contribute to a reduction in the measurement accuracy; therefore the affinity particles used in the method of the present invention have a superior accuracy in selectively capturing only the target protein by using the ligand.

### "Reference example 2"

### "2-aminoethyl methacrylate-N-isopropyl acrylamide-methylene bisacrylamide particles"

1.35 g of N-isopropyl acrylamide and 58 mg of methylene bisacrylamide were added to 200 ml of purified water thoroughly deaerated by means of nitrogen substitution. 7 mg of polymerization initiator V-50 was added, followed by stirring at 70 °C for 30 minutes. 100 mg of 2-aminoethyl methacrylate was added to the mixture, followed by additional stirring at 70 °C for 4 hours; after the temperature was cooled down to room temperature, it was dialyzed in water and lyophilized to obtain the target particles.

### "Phosphorylcholine-modified particles (PC particles (B))"

0.1 g of the 2-aminoethyl methacrylate-N-isopropyl acrylamide-methylene bisacrylamide particles were dispersed in 20 ml of methanol, to which 25 mg of the aldehyde derivative of Synthetic example 1 was added, followed by overnight stirring. 6 mg of sodium cyanoborate was added in an ice water bath, followed by 6 hours of stirring and purification by means of dialysis in water to obtain PC particles (B).

### "Phosphorylcholine-modified particles (PC particles (C))"

0.1 g of the obtained 2-aminoethyl methacrylate-N-isopropyl acrylamide-methylene bisacrylamide particles were dispersed in dimethylsulfoxide (8 ml)-water (2 ml) and 1 ml of water in which 25 mg of the carboxyl derivative of Synthesis example 2, 20 mg of N-hydroxysuccinimide and 23mg of 1-ethyl-3-(3-dimethylaminopropyl) carbodiimide hydrochloride were dissolved was added, followed by overnight stirring. The mixture was purified by means of dialysis in water to obtain PC particles (C).

### "Evaluation of suppression of non-specific adsorption of proteins"

25 mg each of the styrene-glycidyl methacrylate particles prepared in Reference example 1 and PC particles (B) and (C) prepared in Reference example 2 were sampled, to each of which 1 ml of distilled water was added, followed by 1 minute of an ultrasonic treatment. After removing distilled water by means of centrifugation, 1 mL of albumin (100 µg/mL) or lysozyme (100 µ g/mL) was added, followed by a 1 hour reaction at room temperature; after centrifugation (5,000 g), the supernatant was quantified with the Micro BCA method. The results are shown in FIG. 7. The PC particles (B) and (C), which had been treated with phosphorylcholine groups, showed significantly suppressed adsorption of both albumin and lysozyme compared with the styrene-glycidyl methacrylate particles. This indicates that the modification with the phosphorylcholine of formula (1) significantly reduces protein adsorption. Not only agglutination between particles but also the non-specific adsorption of various proteins onto the particles significantly contribute to a reduction in the measurement accuracy; therefore the affinity particles used in the method of the present invention have a superior accuracy in selectively capturing only the target protein by using the ligand.

### "Reference example 3"

### "Phosphorylcholine-modified particles (PC particles (D))"

100 mg of agarose beads (crosslinking ratio 6%) were dispersed in 10 ml of N, N'-dimethylformamide, to which a solution prepared by dissolving and reacting 50 mg of the carboxyl derivative of Synthetic example 2 and 25 mg of thionyl chloride in 1 ml anhydrous N,N'-dimethylformamide was added, followed by 3 hours of stirring at room temperature. Purification by means of successive centrifugation processes first using N,N'-dimethylformamide and then using acetonitrile was done to obtain PC particles (D).

### "Evaluation of suppression of non-specific adsorption of proteins"

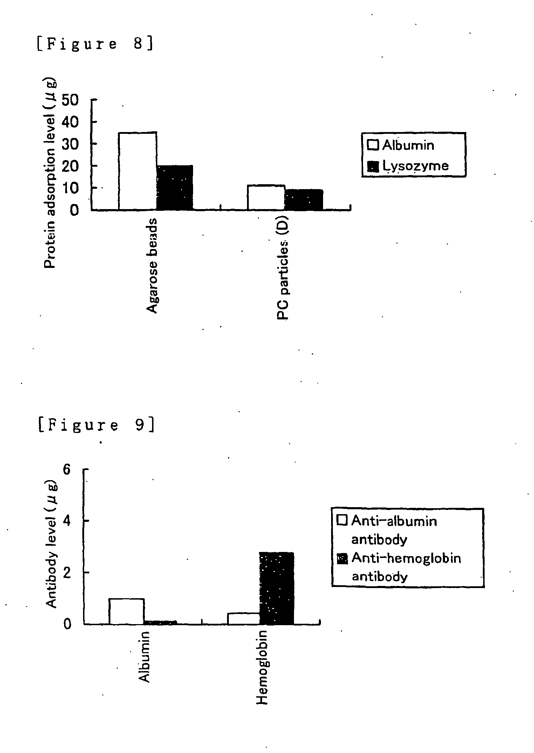

25 mg each of the styrene-glycidyl methacrylate particles prepared in Reference example 1 and PC particles (D) prepared in Reference example 3 were sampled, to each of which 1 ml of distilled water was added, followed by 1 minute of an ultrasonic treatment. After removing distilled water by means of centrifugation, 1 mL of albumin (100 µ g/mL) or lysozyme (100 µ g/mL) was added, followed by a 1 hour reaction at room temperature; after centrifugation (5,000 g), the supernatant was quantified with the Micro BCA method. The results are shown in FIG. 8. The PC particles (D), which had been treated with phosphorylcholine groups, showed significantly suppressed adsorption of both albumin and lysozyme compared with the agarose particles. This indicates that the modification with the phosphorylcholine of formula (1) significantly reduces protein adsorption. Not only agglutination between particles but also the non-specific adsorption of various proteins onto the particles significantly contribute to a reduction in the measurement accuracy; therefore the affinity particles used in the method of the present invention have a superior accuracy in selectively capturing only the target protein by using the ligand.

### "Example 1"

### "Affinity particles"

Next, the affinity separation method shown in claim 6 is described. 10 mg of the aldehyde derivative of Synthetic example 1 was added to 0.1 g of the 2-aminoethyl methacrylate-N-isopropyl acrylamide-methylene bisacrylamide particles obtained in Reference example 2, followed by overnight stirring, after which 3 mg of sodium cyanoborate was added to the mixture in an ice water bath, followed by 6 hours of stirring and dialysis in water to obtain the affinity particles. 1 mL of a glutaraldehyde solution (8%) and 10 mg of sodium cyanotrihydroborate, for stabilizing Schiff base, were added to these affinity particles and the reaction was carried out for 5 hours at room temperature, followed by 5 times of a centrifugation/purification (5,000 g) operation using PBS for cleaning. The affinity particles of claim 2 that have glutaraldehyde as reactive groups or adsorptive groups to which the ligand can bind were thus obtained. 1 mL of bovine albumin (1 mg/mL) or human hemoglobin (1mg/mL) and 10 mg of sodium trihydroborate were added and the reaction was carried out for 1 day at room temperature, followed by 4 times of a centrifugation/purification (5,000 g) with PBS. This bovine albumin or human hemoglobin is the ligand. After this is the affinity separation method shown in claim 7. 1 mL of ethanolamine hydrochloride (0.5 M, pH 7.1) and 10 mg of sodium trihydroborate were added and the reaction was carried out for 1 hour at room temperature to deactivate the remaining glutaraldehyde, followed by 4 times of a centrifugation/purification (5,000 g) with PBS to obtain the affinity particles of claim 3. 1 mL of HRP-conjugated anti-bovine albumin antibody (10 µg/mL) or HRP-conjugated human hemoglobin antibody (10 µg/mL) were added and the reaction was carried out for 1 hour at room temperature, followed by 5 times of a centrifugation/purification (5,000 g) with PBS. An additional 1 mL of PBS was added, followed by stirring; 10 µ1 each was transferred onto a 96-hole well plate and a color development test was conducted using substrate TMBZ; the measurement was done at 450 nm. The results are shown in FIG. 9. The target antibody was captured in a highly selective manner for either ligand used.

### Comparative Example 1

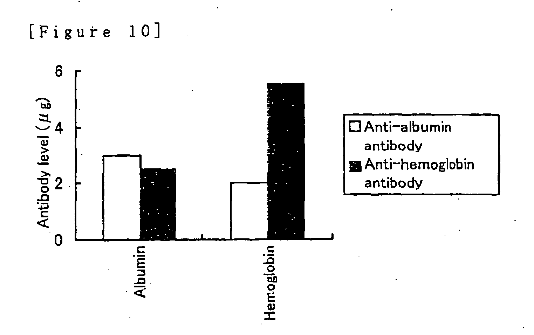

FIG. 10 shows the result of the same operation as Example except for the fact that 0.1 g of the 2-aminoethyl methacrylate-N-isopropyl acrylamide-methylene bisacrylamide particles obtained in Reference example 2 were not modified with phosphorylcholine. The selectivity was shown to be lower compared with Example 1 for either ligand.

### INDUSTRIAL APPLICABILITY

The affinity particles used in the method of the present invention capture only the target substance that is desired to be separated and therefore they exhibit very high selectivity. They also exhibit superior dispersion properties and make separation from liquid samples very easy. Also, less agglutination caused by salts means easy and highly accurate separation of the target substance. Also, they are useful as reagents for the immunoprecipitation method and the latex agglutination method in bio-industries where a highly accurate separation and detection of the target substance is required since they are immune to the influence of salts and capable of highly sensitive detection.

## Claims

1. A method of affinity separation of a target substance by using organic particles that includes (1) a first process whereby arbitrary ligands are bonded to affinity particles that are **characterized by** having phosphorylcholine groups represented by the following formula (1) covalently bonded through amino groups or hydroxyl groups onto the surface of organic particles, 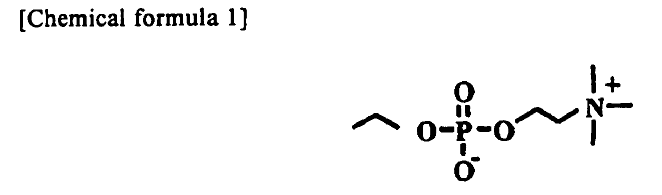 (2) a second process whereby the affinity particles prepared in the first process are dispersed in a liquid sample containing a target substance selectively captured by the arbitrary ligands, and (3) a third process whereby the target substance captured is recovered from the affinity particles.

2. A method of affinity separation according to claim 1 wherein said affinity particles are further **characterized by** having reactive groups or adsorptive groups selected from amino groups and hydroxyl groups, which are capable of bonding with ligands having specific affinity with a certain target substance, covalently bonded or adsorbed onto the surface of organic particles.

3. A method of affinity separation of a target substance by using organic particles that includes (1) a first process whereby affinity particles that are **characterized by** having phosphorylcholine groups represented by the following formula (1) covalently bonded through amino groups or hydroxyl groups onto the surface of organic particles and also by having ligands having specific affinity with a certain target substance covalently bonded or adsorbed through amino groups or hydroxyl groups onto the surface of organic particles are dispersed in a liquid sample containing a target substance selectively captured by the arbitrary ligands, and (2) a second process whereby the target substance captured is recovered from the affinity particles.

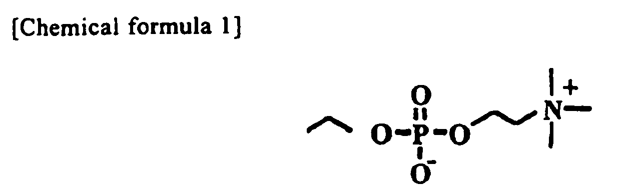

4. The method of claims 1-3 wherein said organic particles are either synthetic particles whose polymers contain one, two, or more types of monomer units chosen from a group consisting of styrene, glycidyl methacrylate, (meth)acrylic acid, N-alkylacrylamide, and alkyl (meth)acrylate, or polysaccharides composed of agarose or sepharose having an average particle size of 20 nm to 500 µm.

5. The method of claims 2-4 wherein said ligands are one, two, or more types of ligands chosen from a group consisting of various antibodies, antigens, enzymes, substrates, receptors, peptides, DNA, RNA, aptamers, protein A, protein G, avidin, biotin, chelating compounds, and various metal ions.

## Patentansprüche

1. Verfahren zur Affinitätstrennung einer Zielsubstanz unter Verwendung organischer Teilchen, welches beinhaltet (1) ein erstes Verfahren, bei dem willkürliche Liganden an Affinitätsteilchen gebunden werden, die **dadurch gekennzeichnet sind, dass** sie Phosphorylcholingruppen der folgenden Formel (1) kovalent über Aminogruppen oder Hydroxylgruppen auf der Oberfläche organischer Teilchen gebunden haben, 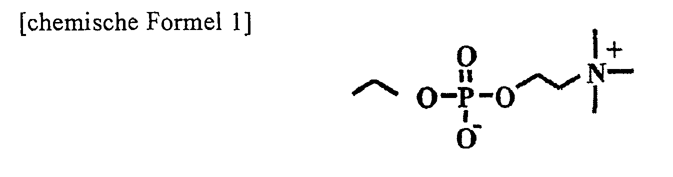 (2) ein zweites Verfahren, bei dem die im ersten Verfahren hergestellten Affinitätsteilchen in einer flüssigen Probe, enthaltend eine selektiv durch die willkürlichen Liganden festgehaltene Zielsubstanz, dispergiert werden, und (3) ein drittes Verfahren, bei dem die festgehaltene Zielsubstanz von den Affinitätsteilchen wiedergewonnen wird.

2. Verfahren zur Affinitätstrennung nach Anspruch 1, wobei die Affinitätsteilchen weiterhin **dadurch gekennzeichnet sind, dass** sie reaktive Gruppen oder adsorptive Gruppen, gewählt aus Aminogruppen und Hydroxylgruppen, die in der Lage sind, an Liganden zu binden, die eine spezifische Affinität zu einer bestimmten Zielsubstanz haben, kovalent auf der Oberfläche organischer Teilchen gebunden oder adsorbiert haben.

3. Verfahren zur Affinitätstrennung einer Zielsubstanz unter Verwendung organischer Teilchen, welches beinhaltet (1) ein erstes Verfahren, bei dem Affinitätsteilchen, die **dadurch gekennzeichnet sind, dass** sie Phosphorylcholingruppen der folgenden Formel (1) kovalent über Aminogruppen oder Hydroxylgruppen auf der Oberfläche von organischen Teilchen gebunden haben und ebenso, dass sie Liganden mit spezifischer Affinität zu einer bestimmten Zielsubstanz kovalent über Aminogruppen oder Hydroxylgruppen auf der Oberfläche von organischen Teilchen gebunden oder adsorbiert haben, in einer flüssigen Probe, enthaltend eine selektiv durch die willkürlichen Liganden festgehaltene Zielsubstanz, dispergiert werden, und (2) ein zweites Verfahren, bei dem die festgehaltene Zielsubstanz von den Affinitätsteilchen wiedergewonnen wird

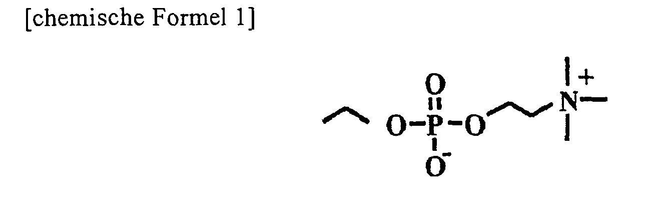

4. Verfahren nach den Ansprüchen 1-3, wobei die organischen Teilchen entweder synthetische Teilchen, deren Polymere einen, zwei oder mehrere Typen an Monomereinheiten enthalten, gewählt aus der Gruppe, bestehend aus Styrol, Glycidylmethacrylat, (Meth)acrylsäure, N-Alkylacrylamid und Alkyl(meth)acrylat, oder Polysaccharide sind, zusammengesetzt aus Agarose oder Sepharose, mit einer durchschnittlichen Teilchengröße von 20 nm bis 500 µm.

5. Verfahren nach den Ansprüchen 2-4, wobei die Liganden ein, zwei oder mehrere Typen von Liganden sind, gewählt aus der Gruppe, bestehend aus verschiedenen Antikörpern, Antigenen, Enzymen, Substraten, Rezeptoren, Peptiden, DNA, RNA, Aptameren, Protein A, Protein G, Avidin, Biotin, Chelatbildungsverbindungen und verschiedenen Metallionen.

## Revendications

1. Procédé de séparation par affinité d'une substance cible en utilisant des particules organiques qui comprend (1) un premier processus dans lequel des ligands arbitraires sont attachés à des particules d'affinité qui sont **caractérisées par le fait qu'**elles possèdent des groupes phosphorylcholine représentés par la formule (1) suivante attachés de manière covalente par le biais de groupes amino ou de groupes hydroxy sur la surface des particules organiques 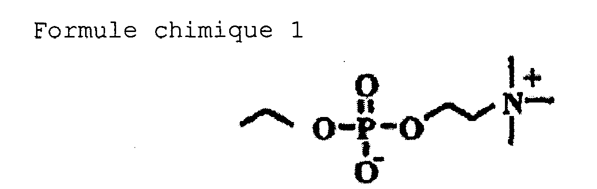 (2) un deuxième processus dans lequel les particules d'affinité préparées dans le premier processus sont dispersées dans un échantillon liquide contenant une substance cible capturée de manière sélective par les ligands arbitraires, et (3) un troisième processus dans lequel la substance cible capturée est récupérée des particules d'affinité.

2. Procédé de séparation par affinité selon la revendication 1, dans lequel lesdites particules d'affinité sont en outre **caractérisées par le fait qu'**elles possèdent des groupes réactifs ou des groupes adsorbants choisis parmi les groupes amino et les groupes hydroxy, qui sont susceptibles de s'attacher à des ligands possédant une affinité spécifique avec une certaine substance cible, attachés de manière covalente ou adsorbés sur la surface des particules organiques.

3. Procédé de séparation par affinité d'une substance cible en utilisant des particules organiques qui comprend (1) un premier processus dans lequel les particules d'affinité, qui sont **caractérisées par le fait qu'**elles possèdent des groupes phosphorylcholine représentés par la formule (1) suivante attachés de manière covalente par le biais de groupes amino ou de groupes hydroxy sur la surface des particules organiques et également **par le fait qu'**elles comprennent des ligands, ayant une affinité spécifique avec une certaine substance cible, attachés de manière covalente ou adsorbés par le biais de groupes amino ou de groupes hydroxy sur la surface des particules organiques, sont dispersées dans un échantillon liquide contenant une substance cible capturée de manière sélective par les ligands arbitraires, et (2) un second processus dans lequel la substance cible capturée est récupérée des particules d'affinité.

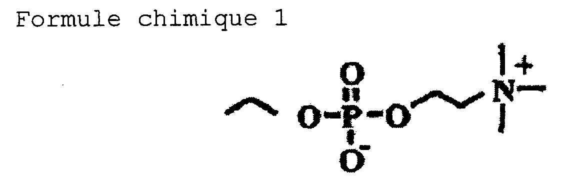

4. Procédé selon l'une quelconque des revendications 1 à 3, dans lequel lesdites particules organiques sont soit des particules synthétiques dont les polymères contiennent un, deux types ou plus de motifs monomères choisis dans le groupe constitué par le styrène, le méthacrylate de glycidyle, l'acide (méth)acrylique, le N-alkylacrylamide et le (méth)acrylate d'alkyle, soit des polysaccharides composés d'agarose ou de sépharose possédant une granulométrie moyenne comprise dans la plage allant de 20 nm à 500 µm.

5. Procédé selon l'une quelconque des revendications 2 à 4, dans lequel lesdits ligands sont un, deux types ou plus de ligands choisis dans le groupe constitué par divers anticorps, antigènes, enzymes, substrats, récepteurs, peptides, l'ADN, l'ARN, les aptamères, la protéine A, la protéine G, l'avidine, la biotine, les composés chélateurs et divers ions métalliques.
